# EUROPEAN PATENT SPECIFICATION

(11) **EP 1 306 073 B1**
(45) Date of publication and mention of the grant of the patent: **16.07.2014**
(21) Application number: 02023805.1
(22) Date of filing: 23.10.2002
(51) Int. Cl.: A61K 8/37, A61K 8/73, A61Q 19/00, A61K 8/34, A61K 8/02, A61K 8/04, D21H 17/64, D21H 17/65, D21H 17/70

(54) **Fibrous web**
Faservlies
Bande fibreuse

(30) Priority: 24.10.2001 JP 2001326224; 17.10.2002 JP 2002303349
(43) Date of publication of application: 02.05.2003
(73) Proprietor: Kawano Paper Co., Ltd., Kochi-shi, Kochi-ken (JP)
(72) Inventor: Taniguchi, Kenji, Hasuda-shi, Saitama-ken (JP)
(74) Representative: Goddar, Heinz J.

(56) References cited:
- WO-A-93/21382
- WO-A-96/08601
- GB-A- 441 347
- US-A- 5 334 286
- US-A- 6 103 060
- DATABASE WPI Week 199329 Derwent Publications Ltd., London, GB; AN 232913 XP002467201 & JP 05 156596 A 9 February 1993 (1993-02-09)
- DATABASE WPI Week 199340 Derwent Publications Ltd., London, GB; AN 317377 XP002467202 & JP 05 229933 A 7 September 1993 (1993-09-07)

## Description

### BACKGROUND OF THE INVENTION

### Field of the Invention

The present invention relates to a fibrous web product and in particular, to a chemically-treated fibrous web product which is formed by subjecting a fibrous web product, such as tissue paper, toilet paper, paper towels, or the like to a chemical treatment, and which is suitably used for sanitary (hygienic) purposes, and which has an improved feel, an improved ability to protect and moisturize the skin, and the like, and to a method of manufacturing the fibrous web product.

### Description of the Related Art

In recent years, the development of various types of fibrous web products has progressed. For example, Japanese Patent Application Laid-Open (JP-A) Nos. 2-224626, 3-900, 4-9121, 4-15021, and the like have proposed, as fibrous web products for sanitary use and other than ordinary tissue paper or toilet paper, various types of chemically-treated fibrous web products which are formed by subjecting the fibrous web of an ordinary tissue paper or toilet paper to a chemical treatment in order to improve the feel thereof and the ability thereof to protect and moisturize the skin.

US-A-5 334 286 relates to tissue papers that comprise a tri-components softener composition comprising a non-ionic softener selected from sorbitan esters, a non-ionic surfactant and a polyhydroxy compound such as Glycerol. The softener is aqueous and is applied by spraying onto the paper.

DATABASE WPI Week 199329 Derwent Publications Ltd., London GB; AN 232913 XP002467201 & JP 05 156596 A 9 February 1993 (1993-02-09) discloses a fibrous web comprising polyhydric alcohol, water retention sizing such as sodium alginate. Polyhydric alcohol and thickening agents, namely sodium alginate, are used.

In GB 441 347 A paper is impregnated with a solution of albuminous adhesive that consists of glue or gelatin and that can comprise a softening agent that consists of glycerine.

Ordinary tissue paper and toilet paper are papers which are formed by producing paper from pulp materials which are the raw material, and thereafter, subjecting the surface thereof to a crepeing processing. Tissue paper and toilet paper differ from one another in that, with tissue paper, wet paper strengthening agents are added at the time of producing the paper in order to improve the water resistance, whereas with toilet paper, no wet paper strengthening agent is added.

The fibers of such fibrous webs for sanitary purposes are rubbed by the crepeing processing, such that the paper becomes so-called bulky paper. Bulky paper has an excellent water absorbing ability and an excellent water retaining ability, but has the problems that, because it is formed only from pulp fibers, the feel thereof is insufficient and repeated use thereof may irritate or abrade the skin.

For these reasons, with regard to tissue paper for example, chemically-treated fibrous web products for sanitary use such as the following have been proposed: JP-A No. 5-156596 discloses a moisture retaining paper containing various types of moisture retaining components, such as glycerin and the like, in a fibrous web, and Japanese Patent Application National Publication No. 9-506682 discloses a lotioned tissue in which a wax-based oil or the like is coated onto the surface of a fibrous web. However, among such chemically-treated fibrous webs for sanitary use, in a moisture retaining paper, the pulp becomes plastic and flexible due to the contained moisture retaining component absorbing and retaining moisture from the environmental atmosphere. Thus, the dampness, pliability, and low irritability to the skin of such products is excellent. However, there are problems in that, due to the high moisture content, there is the tendency for a sticky sensation to arise, the downy tactile sensation is insufficient, the strength tends to deteriorate, and such products are not suited to applications in which they are strongly rubbed. Moreover, the tactile sensation is influenced by the amount of contained moisture, which varies depending on the ambient temperature and humidity as well as the season. It is therefore difficult to stabilize the tactile sensation of the product.

Moreover, the feel of lotioned tissues is insufficient, and a wax-based oil is coated on the surface of the fibrous web. Therefore, a problem arises in that, when such lotioned tissues are used, the oil moves onto the skin and creates a greasy sensation.

### SUMMARY OF THE INVENTION

The present invention overcomes the above-described drawbacks of the conventional art, and aims to achieve the following objects. Namely, an object of the present invention is to provide a fibrous web product which is suited to sanitary (hygienic) uses, and which, while maintaining properties such as sufficient strength in actual use, water absorbing ability, water retaining ability and the like, also stably realizes a tactile sensation and feel which balance smoothness, pliability, downiness and the like which heretofore were unable to be realized in conventional fibrous web products, and which has excellent properties of low irritability to skin and the effect to protect and moisturize the skin, and which has particularly excellent downiness and smoothness without a sticky sensation or a greasy feeling. The objects of the present invention is achieved by a fibrous web product according to claim 1.

Preferably the water-containing gel composition is thermally reversible, such that it is set in a gel state by being cooled and is set in a sol state by being heated.

The water-containing gel composition further may contain silk powder.

The water-containing gel composition may be a foamed gel composition having a finely porous structure.

Advantageously, the foamed gel composition which has the finely porous structure is formed by foaming of the gas being generated due to reaction of the foaming agent and the acid when the gel composition containing a framing agent is made to contact an acid.

Alternatively, the foamed gel composition which has the finely porous structure may be formed by foaming of the gas being generated by heating a gel composition containing a foaming agent decomposing the foaming agent.

The foaming agent may be at least one type selected from the group consisting of sodium hydrogencarbonate, ammonium carbonate, azodicarbonamide, and benzene sulfonyl hydrazide. Preferably the fibrous web contains pulp fibers, and the pulp fibers contain cross-linked pulp fibers in an amount of from 1% by mass to 80% by mass.

### DESCRIPTION OF THE PREFERRED EMBODIMENTS

Hereinafter, the fibrous web product according to special embodiments of the present invention will be described.

### (Fibrous Web Product)

The fibrous web product of the present invention is a fibrous web product in which a water-containing gel composition is contained in a fibrous web. The water-containing gel composition includes a moisture retaining component, a gelling agent, and water. The water is at least one of water retained by the moisture retaining component and water absorbed from the atmosphere by the moisture retaining component.

### <Fibrous Web>

The fibrous web product of the present invention contains a water-containing gel composition in a fibrous web. Paper and non-woven fabrics are preferable as the fibrous web. Suitable examples of the raw material of the paper are wood fibers (coniferous trees, broad-leaved trees), non-wood vegetable plant fibers (linen, bast fiber, arboreous cotton, straw, and the like), rayon fibers, and the like, as well as cross-linked fibers obtained by cross-linking the above-listed fibers. Suitable examples of the raw material of the non-woven fabric are wood pulp fibers, natural fibers (arboreous cotton, wool, silk, and the like), semi-synthetic fibers (rayon and the like), synthetic fibers (nylon, polyethylene, polyester, polypropylene, and the like), as well as cross-linked fibers obtained by cross-linking the above-listed fibers. Any of these raw materials may be used.

When such cross-linked fibers are compounded in the fibrous web, the compounding ratio thereof is preferably 1% by mass to 80% by mass, and a compounding ratio of 20% by mass to 60% by mass is more preferable. If the compounding ratio is less than 1% by mass, it is nearly impossible to obtain the desired bulk, whereas if the compounding ratio exceeds 80% by mass, the strength of the paper deteriorates extremely.

In the fibrous web product of the present invention, an aspect in which the fibrous web contains pulp fibers and the pulp fibers contain cross-linked pulp fibers in an amount of 1% by mass to 80% by mass is particularly preferable. By using, as the fibrous web, a bulky base paper using cross-linked pulp fibers, the tactile sensation, such as the downiness and the like, of the fibrous web product of the present invention can be further improved.

### <Water-Containing Gel Composition>

The fibrous web product of the present invention contains a water-containing gel composition in the fibrous web. The water-containing gel composition preferably is in a gel state at ordinary temperature. Further, the water-containing gel composition is preferably thermally reversible such that it is set in a gel state when cooled and is set in a sol state when heated. Note that, in the present specification, "ordinary temperature" means 15 DEG C to 25 DEG C in JIS K0211, K00500.

In the fibrous web product of the present invention, by including a water-containing gel composition in the fibrous web, the feel of the product, such as the smoothness, the pliability, the downiness and the like thereof, is improved. By maintaining the gel state at ordinary temperature at which the product is used, the above-described preferable properties can be stably realized. Further, from the standpoint of ease of impregnating the water-containing gel composition into the fibrous web, it is preferable to use a water-containing gel composition which is thermally reversible.

Further, it is more preferable that the water-containing gel composition is a foamed gel composition which has a finely porous structure. Particularly preferable are an aspect in which the foamed gel composition which has a finely porous structure is formed by making an acid and a gel composition containing a foaming agent contact one another such that gas is generated by the reaction of the foaming agent and the acid, and the foamed gel composition is formed by the foaming of the gas; and an aspect in which the foamed gel composition which has a finely porous structure is formed by heating a gel composition containing a foaming agent, and gas is generated by the decomposition of the foaming agent, and the foamed gel composition is formed by the foaming of the gas.

By using, as the water-containing composition, a foamed gel composition having, at the interior thereof, spaces formed by foaming, i.e., a foamed gel composition having a finely porous structure, the properties of the fibrous web product of the present invention, such as pliability, downiness, and the like, can be improved even more.

In the fibrous web product of the present invention, the contained amount of the water-containing gel composition is, when the fibrous web is paper, preferably 0.1 to 100% by mass with respect to the fibrous web. If the contained amount of the water-containing gel composition is less than 0.1 % by mass with respect to the fibrous web, there is no improvement in the tactile sensation such as the softness, the pliability, the downiness and the like. The contained amount of the water-containing gel composition exceeding 100% by mass does not result in any further effects and is therefore uneconomical, and a sticky sensation arises and the strength deteriorates.

Therefore, for fibrous web products whose fibrous webs are paper, by making the contained amount of the water-containing gel composition fall within the range of 0.1 to 100% by mass with respect to the fibrous web, the various types of tactile sensations can be improved as compared with the conventional art, and strength which is sufficient in practice can be obtained.

In particular, if the contained amount of the water-containing gel composition falls within the range of 10 to 50% by mass with respect to the fibrous web, the tactile sensations of softness, pliability, downiness and the like can be improved even more while the overall balance thereof is maintained. Even more preferably, the contained amount of the water-containing gel composition is 20 to 30% by mass.

If the fibrous web is a non-woven fabric, the contained amount of the water-containing gel composition is preferably 0.5 to 200% by mass with respect to the fibrous web. If the contained amount is less than 0.5% by mass with respect to the fibrous web, there is no improvement in the tactile sensations, and if the contained amount exceeds 200% by mass, no further effects are achieved.

In order to further improve the balance of the tactile sensations, the contained amount of the water-containing gel composition is preferably in a range of 10 to 100% by mass, and most preferably 30 to 50% by mass.

In the fibrous web product of the present invention, the water-containing gel composition contains a moisture retaining component, a gelling agent, and water. The water is at least one of water retained by the moisture retaining component and water absorbed from the atmosphere by the moisture retaining component.

In the water-containing gel composition, it is preferable that the contained amount of the moisture retaining component is 20 to 95% by mass, the contained amount of the gelling agent is 0.01 to 30% by mass, and the contained amount of the water is 5 to 70% by mass.

### -Moisture Retaining Component and Water-

The moisture retaining component is glycerin

The moisture retaining component retains the moisture provided to the water-containing gel composition and, depending on the environmental conditions, absorbs moisture from the environmental atmosphere, so as to increase the amount of moisture contained in the fibrous web. In this way, the fibers swell, the hydrogen bonds between fibers are relaxed, the resistance to external force decreases, and the contained moisture serves as a lubricant between the fibers and the object (i.e., skin or the like).
from the standpoint of increasing the moisture absorbing ability of the fibrous web.

The mass ratio (when the absorption of moisture has reached equilibrium) of the glycerin and the moisture which the glycerin retains and/or absorbs in a usual daily-life environment is 10: 2-7.

It can be thought that the relationship (when the absorption of moisture has reached equilibrium) between the moisture retaining component contained in the water-containing gel composition and the moisture which the moisture retaining component retains and/or absorbs from the atmosphere, is determined in accordance with the amount of moisture in the environmental atmosphere (or the humidity), the moisture absorbing ability of the moisture adsorbing component itself, and the like. Therefore, in a dry environment, the amount of moisture which is retained and/or absorbed is low with respect to the moisture retaining component, and in a high-humidity environment, the amount of moisture which is retained and/or absorbed is high with respect to the moisture retaining component.

In the fibrous web of the present invention, when the absorption of moisture from the atmosphere by the moisture retaining component has reached equilibrium, the amount of moisture contained in the water-containing gel composition, and the amount of moisture which is retained and/or absorbed from the atmosphere by the moisture retaining component, are equal.

However, due to changes in environmental conditions, such as changes in the humidity or the like, there are cases in which a non-equilibrium state temporarily arises, and the amount of moisture contained in the water-containing gel composition fluctuates. In these cases as well, the above-mentioned amount of moisture is preferably within a range of 50% by mass to 200% by mass of the amount of moisture which the moisture retaining component absorbs from the atmosphere when equilibrium has been reached after environmental changes, and is more preferably within a range of 70% by mass to 140% by mass, and is particularly preferably within a range of 90% by mass to 110% by mass.

If the amount of moisture contained in the water-containing gel composition is less than 50% by mass of the amount of moisture which the moisture retaining components retains and/or absorbs from the atmosphere when equilibrium has been reached after environmental clanges, in a case in which the amount of moisture in the atmosphere is low such as when it is dry or the like, the water-containing gel composition hardens and the tactile sensation deteriorates, which is not preferable. On the other hand, if the amount is greater than 200% by mass, in a case in which the amount of moisture in the atmosphere is high such as when there is high humidity or the like, the water-containing gel composition becomes too soft and the strength of the fibrous web itself deteriorates, which is not preferable.

Further, the aforementioned ranges are also preferable because the fibrous web of the present invention is often stored in a state in which it is placed in a packaging container, such as a paper box or film or the like, which is not completely sealed, and the fibrous web of the present invention therefore is always affected by the environmental atmosphere.

### -Gelling Agent-

The water-containing gel composition of the present invention contains a gelling agent. The contained amount of the gelling agent in the water-containing gel composition is preferably 0.01 to 30% by mass.

At least one type selected from the group consisting of hexaglycerin monostearate and agar used as the gelling agent.

These gelling agents impart a tactile sensation in which smoothness, pliability, downiness and the like are well-balanced.

As the water-containing gel composition containing the gelling agent, a substance, which is thermally reversible such that it gels upon cooling and liquifies (is set in a sol state) upon heating, easily permeates into the fibrous web. In this case, a substance is used which, if the temperature falls once to the gel freezing point or below, is in a gel state at an ordinary temperature (15 DEG C to 25 DEG C in JIS K0211, K0050) of less than or equal to the gel melting point.

The gelling agent gels the moisture retaining component and the water which have permeated into the fibrous web, and causes formation of the water-containing gel composition having a three-dimensional network structure, and exhibits elasticity, and imparts downiness to the entire fibrous web. Further, as compared with a conventional liquid composition containing a moisture retaining component and water, the fibrous web product relating to the present invention has a mesh structural body due to the gelling. Thus, breaking away of the fibers can be suppressed, and deterioration in the paper strength and generation of paper dust, which are caused by the breakage of hydrogen bonds between fibers, can be suppressed.

In the water-containing gel composition, it is preferable that the contained amount of the moisture retaining component is 20 to 95% by mass, the contained amount of the gelling agent is 0.01 to 30% by mass, and the contained amount of the water is 5 to 70% by mass.

When glycerin is used as the moisture retaining component and agar or hexaglycerin monostearate is used as the gelling agent, the compounding mass ratio of the glycerin, the water, and the agar or hexaglycerin monostearate in the water-containing gel composition is, for example, 10 : 2-7 : 0.01-1.

### -Other Components-

In order to improve the smoothness of the fibrous web, silicones such as amino-modified silicone oil, epoxy-modified silicone oil, carboxy-modified silicone oil, polyether-modified silicone oil, dimethyl polysiloxane, and the like, or silk powder or the like may be contained in the water-containing gel composition.

If a saturated fatty acid or salt thereof, such as triethanolamine stearate, is also included, the downiness is improved even more. The chain length of the saturated fatty acid is preferably C12 to C22. Further, a saturated aliphatic alcohol or a saturated fatty acid ester may be used.

As needed, a preservative/fungicide such as p-hydroxybenzoic acid ester may be added to the water-containing gel composition.

### -Foamed Gel Composition-

In the fibrous web product of the present invention, the water-containing gel composition is preferably a foamed gel composition having a finely porous structure.

The foamed gel composition having a finely porous structure is particularly preferably formed by making an acid and a gel composition containing a foaming agent contact one another such that gas is generated by the reaction of the foaming agent and the acid, and the foamed gel composition is formed by the foaming of the gas.

By using a foamed gel composition having, at the interior thereof, spaces due to foaming, i.e., having a finely porous structure, as the water-containing gel composition, the properties such as the pliability, downiness and the like of the fibrous web product of the present invention can be improved even more.

The method of including the foamed gel composition in the fibrous web as the water-containing gel composition will be described later.

The fibrous web product of the present invention is suitably used as a fibrous web product for various sanitary uses such as tissue paper, toilet paper, paper towels, non-woven fabric type tissues, baby-wipes for infants, webs for makeup, and other applications for directly contacting the skin.

### (Method of Manufacturing Fibrous Web Product)

The method of manufacturing the fibrous web product of the present invention by making a fibrous web contain a water-containing gel composition will be described hereinafter.

### (1) Fibrous Web Prepared by Wet-Type Manufacturing Method

In the process of manufacturing the fibrous web, in the damp state before formation of the web, the water-containing gel composition is applied to and impregnated in by spraying or the like, or, after the fibrous web has been prepared and dried, the water-containing gel composition is applied to and impregnated in by spraying or by printing rollers or the like.

When the water-containing gel composition is applied to and impregnated in by spraying, the water-containing gel composition is used in a liquid form in which it is diluted by or dispersed in water. In the case of a water-containing gel composition which is thermally reversible, the water-containing gel composition is used in a liquid (sol) state by being heated once to the gel melting point or more and then the temperature thereof being maintained at at or above the gel freezing point.

If the water-containing gel composition is diluted by water, the excess moisture used in dilution is heated and evaporated by a drier of a paper mashine if in the midst of the paper manufacturing process. Or, if after the paper manufacturing process, the fibrous web is passed through a drier or the like such that the excess moisture is evaporated.

When a thermally reversible water-containing gel composition is impregnated in the fibrous web, after impregnation, the fibrous web is cooled to at or below the gel freezing point.

After the fibrous web has been manufactured and dried, if the water-containing gel composition is to be impregnated therein by printing rollers or the like, the water-containing gel composition is coated thereon as is. Or, in the case of a thermally reversible water-containing gel composition, the composition may be coated in a liquid (sol) state by once being heated to the gel melting point or higher, and then the temperature thereof being maintained at or above the gel freezing point. In this case, after the coating, the fibrous web is cooled to the gel freezing point or lower.

In any case, if needed, the amount of moisture (the degree of wetness) of the water-containing gel composition, which has been applied to and impregnated in the fibrous web, is adjusted by utilizing the amount of water in the atmosphere of the environment.

If the water-containing gel composition is a foamed gel composition having a finely porous structure, following methods i) through iii) are suitable examples.
i) A method in which a foaming agent is contained in the gel composition, and the gel composition is coated on and impregnated in the fibrous web. Thereafter, by heating, the foaming agent decomposes and foams such that a foamed gel composition, which has a finely porous structure, is formed within the fibrous web.
ii) A method in which a gel composition including a foaming agent is coated on one surface of a fibrous web which is a single layer or is formed by superposing plural layers, and on the other surface, a gel composition containing an acid is coated. Thereafter, by winding the fibrous web in a roll-form, the gel composition containing the foaming agent and the gel composition containing the acid, which have been coated on the fibrous web, are made to contact one another. Due to the foaming of the gas generated by the reaction of the foaming agent and the acid, a foamed gel composition which has a finely porous structure is formed within the fibrous web.
iii) A method in which a gel composition containing a foaming agent is coated on the surface so as to be impregnated in a fibrous web which is a single layer or is formed by superposing plural layers. Thereafter, by applying an acid to the surface of the fibrous web so as to impregnate the acid, the foaming agent and the acid react. Due to the foaming of the generated gas, a foamed gel composition which has a finely porous structure is formed within the fibrous web.

In above method iii), an aspect is preferable in which the method of applying the acid is the application of the acid by spraying or the like, uniformly and in a mist-like form, onto the surface of the fibrous web.

The foaming agent used in above methods i) through iii) is not particularly limited provided that it generates a gas by being heated or by reacting with an acid. However, inorganic foaming agents such as sodium hydrogencarbonate, ammonium carbonate, and the like, and organic foaming agents such as azodicarbonamide, benzene sulfonyl hydrazide and the like are suitably used.

Further, the acid used in above methods ii) and iii) is not particularly limited provided that it generates a gas by reacting with the foaming agent. However, lactic acid, citric acid and the like are preferably used from the standpoint of stability.

Among combinations of the foaming agent and the acid, combinations such as sodium hydrogencarbonate and lactic acid, and sodium hydrogencarbonate and citric acid, and the like are preferable in the fibrous web of the present invention.

Further, the gas, which is generated by the foaming agent foaming by being heated or by reacting with an acid, differs in accordance with the type of the foaming agent and the type of acid. However, from the standpoint of the stability during manufacturing, it is preferable that the gas is carbon dioxide.

### (2) Fibrous Web Prepared by Dry-Type Manufacturing Method

After the fibrous web is formed, a processing solution is applied (coated/impregnated or the like) thereto by spraying or by printing rollers or the like, and the same processings as those described above are carried out.

Among the methods of manufacturing the fibrous web product of the present invention, the following aspects, in which a foamed gel composition having a finely porous structure is contained as a water-containing gel composition in a fibrous web, are particularly preferably used.

A first aspect of the method of manufacturing a fibrous web product of the present invention is a method of manufacturing a fibrous web product containing a water-containing gel composition in a fibrous web, the method comprising following steps a through c.

Step a is a step of coating a gel composition, which contains a foaming agent, on one surface of a fibrous web which is one layer or is formed by superposing plural layers. Step b is a step of coating a gel composition, which contains an acid, on another surface of the fibrous web which is one layer or is formed by superposing plural layers. Step c is a step of forming, within the fibrous web, the water-containing gel composition which is a foamed gel composition having a finely porous structure, by winding the fibrous web in a roll form, making the gel composition containing the foaming agent and the gel composition containing the acid, which have been coated on the fibrous web, contact each other, and due to foaming of gas generated by reaction of the foaming agent and the acid, forming the water-containing gel composition within the fibrous web.

Further, a second aspect of the method of manufacturing a fibrous web product of the present invention is a method of manufacturing a fibrous web product containing a water-containing gel composition in a fibrous web, the method comprising following steps a and b.

Step a is a step of coating and impregnating a gel composition, which contains a foaming agent, at the surface of a fibrous web which is one layer or is formed by superposing plural layers. Step b is a step of forming the water-containing gel composition, which is a foamed gel composition having a finely porous structure, within the fibrous web by making the foaming agent and an acid react by applying and impregnating the acid at the surface of the fibrous web at which the gel composition, which contains the foaming agent, was coated and impregnated, and due to foaming of generated gas, foaming the water-containing gel composition within the fibrous web.

Further, a third aspect of the method of manufacturing a fibrous web product of the present invention is a method of manufacturing a fibrous web product containing a water-containing gel composition in a fibrous web, the method comprising following steps a and b.

Step a is a step of coating and impregnating a gel composition, which contains a foaming agent, at a surface of a fibrous web which is one layer or is formed by superposing plural layers. Step b is a step of forming a water-containing gel composition which is a foamed gel composition having a finely porous structure, within the fibrous web by decomposing the foaming agent by heating the fibrous web at which the gel composition, which contains the foaming agent, was coated and impregnated, and due to foaming of generated gas, forming the water-containing gel composition within the fibrous web.

### EXAMPLES

Hereinafter, the present invention will be described on the basis of Examples except for Example 7 that is not part of the invention. However, the present invention is not to be limited to these

### Examples.

### 1) Example 1

Example 1 is a case in which paper is used as the fibrous web.

First, a pulp, in which were compounded 80% by mass of NBKP (a coniferous tree kraft process bleached pulp) and 20% by mass of LBKP (a broad-leaved tree kraft process bleached pulp), was refined to a freeness of 630 to 640 ml (CSF). A wet paper strengthening agent was mixed into the pulp fibers in an amount of 0.15% by mass with respect to the pulp fibers, such that a base paper for sanitary paper having a basis weight of 14.0 g/m<2> and a crepeing rate of 18% was prepared.

On the other hand, processing liquids were prepared by heating, dissolving and mixing together the respective components listed for Examples 1 through 10 and Comparative Examples 1 through 5 of Table 1. The processing liquid was coated on and impregnated into both surfaces of a two-ply structure of the base paper, by using a gravure printing roller. Note that the numbers in Table 1 are percentages by mass with respect to the base paper.

Here, in Examples 1 through 10, the processing liquid was coated in a liquid (sol) state obtained by heating the processing liquid once to the gel melting point or more, and then maintaining the temperature thereof at the gel freezing point or higher. Next, the coated base paper was cooled to less than or equal to the gel freezing point so as to gel the processing liquid.

In Comparative Example 4, the processing liquid was coated after being heated to and held at the melting point of paraffin or higher.

Comparative Example 5 is an untreated base paper for sanitary paper.

Next, each of the respective base papers, which had been impregnated with the processing liquids of Examples 1 through 10 and Comparative Examples 1 through 5 of Table 1, was left to stand and stabilized for 20 hours at a humidity of 65 +/- 10% and a temperature of 20 +/- 5 DEG C. Thereafter, the base paper was processed into tissue paper form (a two-ply structure which was 200 mm long and 225 mm wide), and respective measurements which will be described later (function tests, measurement of the amount of paper dust, and physical tests) were carried out thereon.

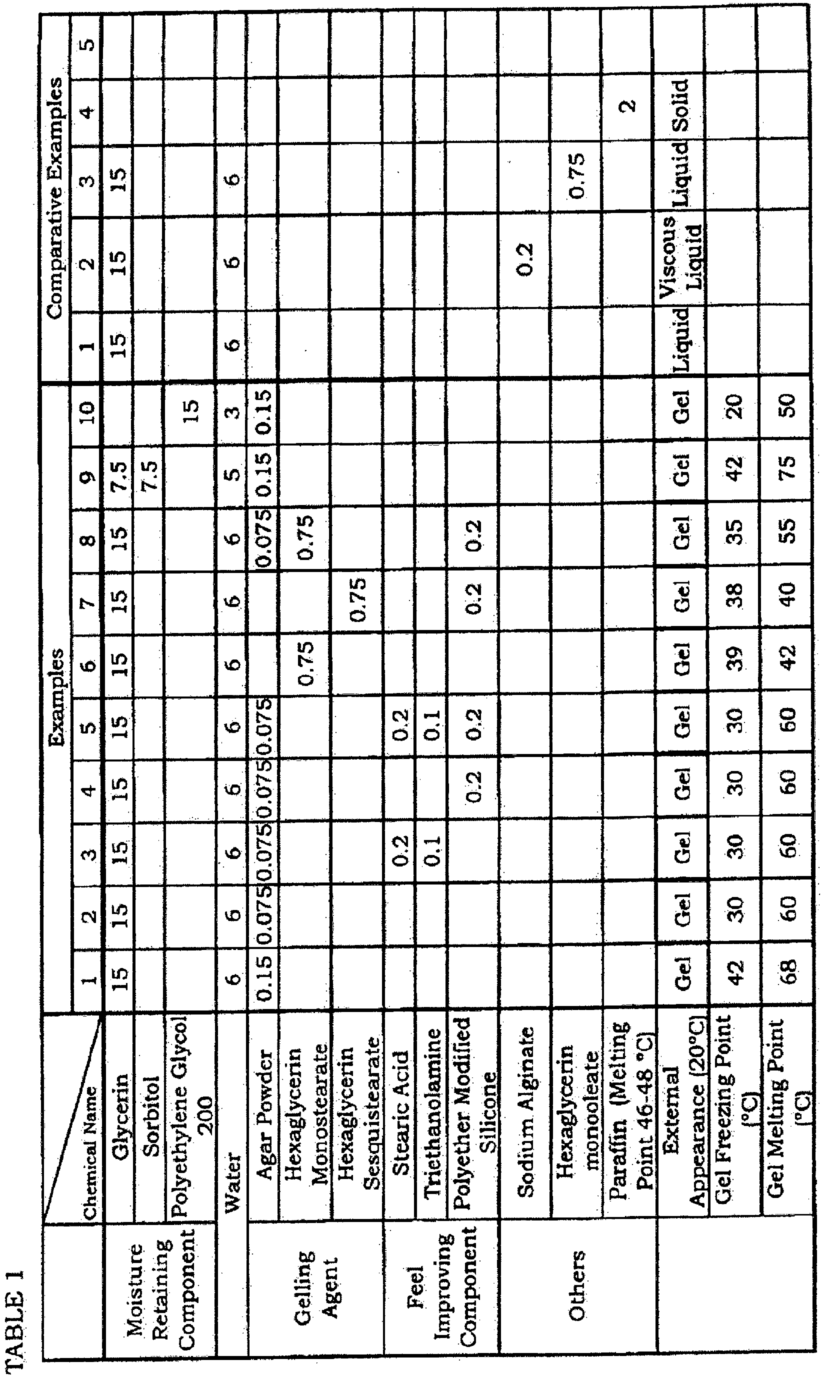

### 2) Example 2

This Example is a case in which a non-woven fabric is used as the fibrous web.

First, a 200 mm long and 225 mm wide section of a dry-type pulp non-woven fabric (product name "Kinocloth", manufactured by Oji Kinocloth Co., Ltd.) having a basis weight of 40 g/m<2> was cut. Next, processing liquids were prepared by heating, dissolving and mixing together the respective components listed in Table 2. The processing liquid was coated on and impregnated into both surfaces of a two-ply sample, by using a gravure printing roller. Note that the numbers in Table 2 are percentages by mass with respect to the non-woven fabric.

Here, in Examples 11 through 15, the processing liquid was coated in a sol state obtained by heating the processing liquid once to the gel melting point or more, and then maintaining the temperature thereof at the gel freezing point or higher. Next, the coated base paper was cooled to less than or equal to the gel freezing point so as to gel the processing liquid.

Comparative Example 9 is an untreated dry-type pulp non-woven fabric.

Thereafter, for each of the materials in which the processing liquids of Examples 11 through 15 and Comparative Examples 6 through 8 of Table 2 were impregnated, and for Comparative Example 9 as well, the sample was, in the same way as in Example 1, left to stand and stabilized for 2 hours at a humidity of 65 +/- 10% and a temperature of 20 +/- 5 DEG C. Thereafter, the sample was subjected to respective measurements (function tests) which will be described later.

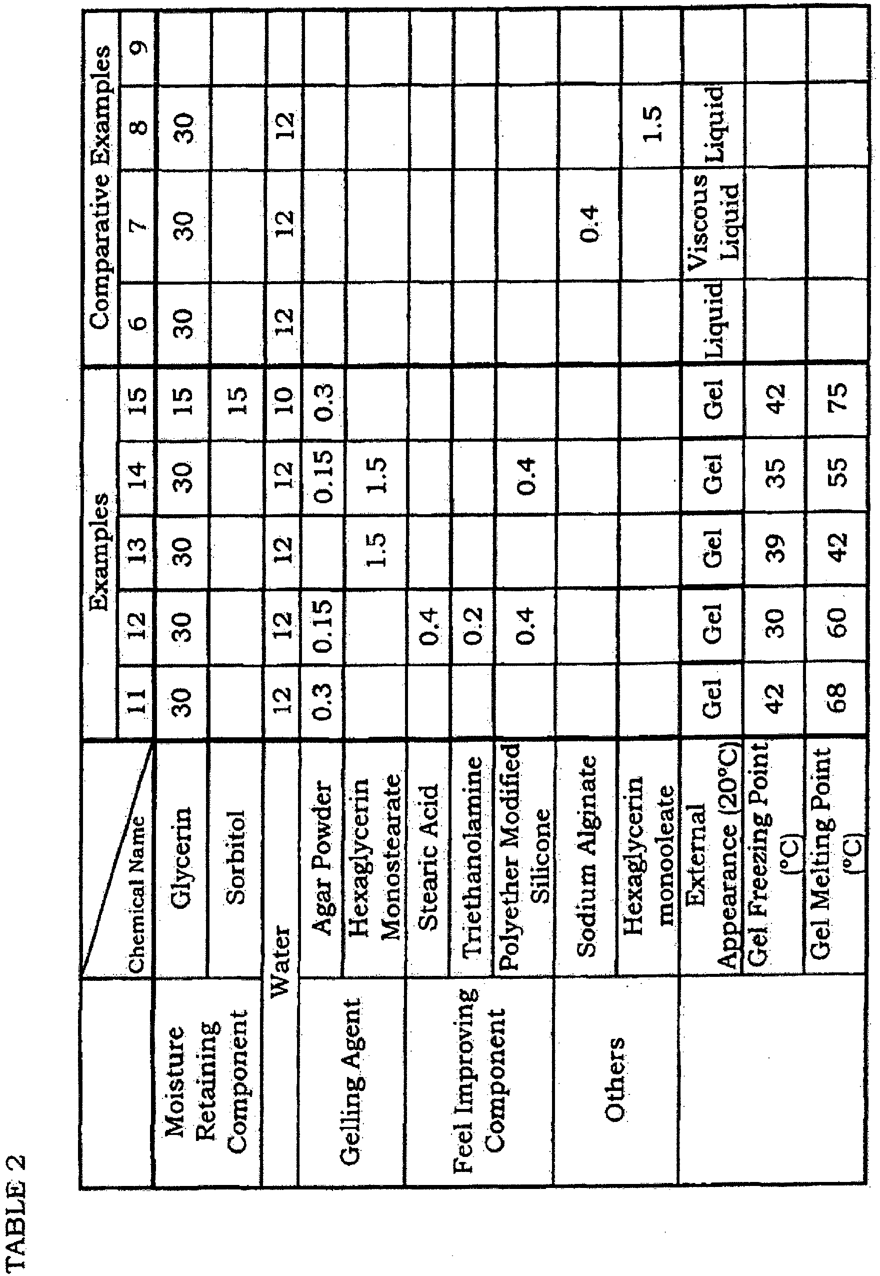

### 3) Example 3

Example 3 is a case in which paper is used as the fibrous web, and a foamed gel composition is used as the water-containing gel composition.

First, a pulp, in which were compounded 80% by mass of NBKP (a coniferous tree kraft process bleached pulp) and 20% by mass of LBKP (a broad-leaved tree kraft process bleached pulp), was refined to a freeness of 630 to 640 ml (CSF). A wet paper strengthening agent was mixed into the pulp fibers in an amount of 0.15% by mass with respect to the pulp fibers, such that a base paper for sanitary paper having a basis weight of 14.0 g/m<2> and a crepeing rate of 18% was prepared.

On the other hand, processing liquids were prepared by heating, dissolving and mixing together the respective components listed under 16-A and 17-A in Examples 16 and 17 of Table 3. The processing liquid was coated on one surface of a two-ply structure of the base paper, by using a gravure printing roller. Further, processing liquids were prepared by heating, dissolving, and mixing together the respective components listed under 16-B and 17-B, and the processing liquid was coated on the opposite surface. Next, by winding the coated base paper into a roll form, the processing liquids contacted each other such that a foamed gel composition, which had fine pores at the interior thereof, was generated within the fibrous web.

Further, processing liquids were prepared by heating, dissolving and mixing together the respective components listed under 18-A and 19-A in Examples 18 and 19 of Table 3. The processing liquid was coated on and impregnated into both surfaces of a two-ply structure of the base paper, by using a gravure printing roller. Next, the liquid of 18-B or 19-B was sprayed on this base paper, such that a foamed gel composition, which had fine pores at the interior thereof, was generated within the fibrous web. Note that the numbers in Table 3 are percentages by mass with respect to the base paper.

Thereafter, each of the samples, in which a foamed gel composition was generated within the fibrous web by using the processing liquids of Examples 16 through 19 of Table 3, was left to stand and stabilized for 20 hours at a humidity of 65 +/- 10% and a temperature of 20 +/- 5 DEG C, in the same way as in Examples 1 and 2. Thereafter, the sample was subjected to respective measurements (function tests) which will be described later.

### 4) Example 4

Example 4 is a case in which paper is used as the fibrous web, and a silk powder is compounded into the water-containing gel composition.

First, a pulp, in which were compounded 80% by mass of NBKP (a coniferous tree kraft process bleached pulp) and 20% by mass of LBKP (a broad-leaved tree kraft process bleached pulp), was refined to a freeness of 630 to 640 ml (CSF). A wet paper strengthening agent was mixed into the pulp fibers in an amount of 0.15% by mass with respect to the pulp fibers, such that a base paper for sanitary paper having a basis weight of 14.0 g/m<2> and a crepeing rate of 18% was prepared.

On the other hand, processing liquids were prepared by heating, dissolving and mixing together the respective components of Examples 20 and 21 of Table 3. The processing liquid was coated on and impregnated into both surfaces of a two-ply structure of the base paper, by using a gravure printing roller. Note that the numbers in Table 3 are percentages by mass with respect to the base paper.

Here, in Examples 20 and 21, the processing liquid was coated in a liquid (sol) state obtained by heating the processing liquid once to the gel melting point or more, and then maintaining the temperature thereof at the gel freezing point or higher. Next, the coated base paper was cooled to less than or equal to the gel freezing point so as to gel the processing liquid.

Thereafter, each of the samples, into which the processing liquids of Examples 20 and 21 of Table 3 were impregnated, was left to stand and stabilized for 20 hours at a humidity of 65 +/-10% and a temperature of 20 +/- 5 DEG C, in the same way as in Examples 1 through 3. Thereafter, the sample was subjected to respective measurements (function tests) which will be described later.

### 5) Example 5

Example 5 is a case in which a bulky paper is used as the fibrous web.

First, a pulp, in which were compounded 80% by mass of NBKP (a coniferous tree kraft process bleached pulp) and 20% by mass of LBKP (a broad-leaved tree kraft process bleached pulp), was refined to a freeness of 630 to 640 ml (CSF). 60% by mass of this pulp fiber and 40% by mass of a cross-linked pulp fiber (HBA-FF manufactured by Weyerhaeuser Company) were compounded together so as to prepare a raw material pulp fiber. A wet paper strengthening agent was mixed into the pulp fibers in an amount of 0.3% by mass with respect to the pulp fibers, such that a base paper for sanitary paper having a basis weight of 14.0 g/m<2> and a crepeing rate of 18% was prepared.

On the other hand, processing liquids were prepared by heating, dissolving and mixing together the respective components listed for Examples 22 and 23 of Table 3. The processing liquid was coated on and impregnated into both surfaces of a two-ply structure of the base paper, by using a gravure printing roller. Note that the numbers in Table 3 are percentages by mass with respect to the base paper.

Here, in Examples 22 and 23, the processing liquid was coated in a liquid (sol) state obtained by heating the processing liquid once to the gel melting point or more, and then maintaining the temperature thereof at the gel freezing point or higher. Next, the coated base paper was cooled to less than or equal to the gel freezing point so as to gel the processing liquid.

Comparative Example 10 is an untreated base paper for sanitary paper.

Thereafter, for each of the samples into which the processing liquids of Examples 22 and 23 in Table 3 had been impregnated, and for the sample of Comparative Example 10, the sample was left to stand and stabilized for 20 hours at a humidity of 65 +/- 10% and a temperature of 20 +/- 5 DEG C, in the same way as in Examples 1 through 4. Thereafter, the sample was subjected to respective measurements (function tests) which will be described later.

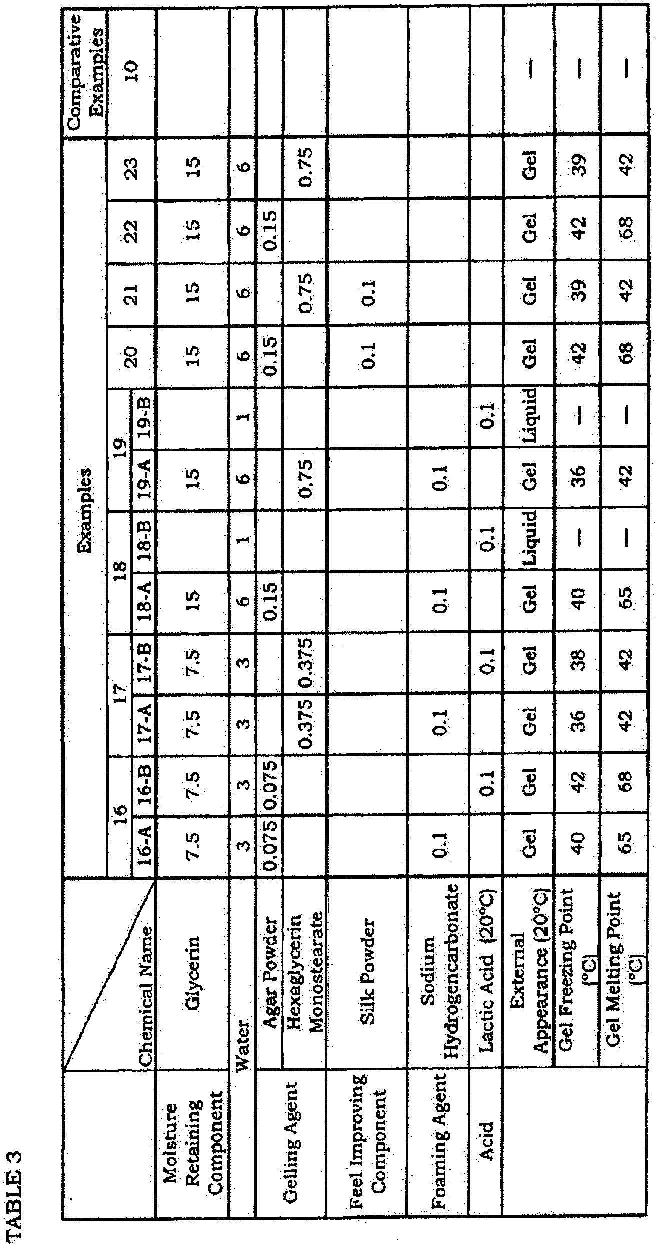

Note that, in Tables 1, 2, and 3, each of the samples of the Examples contains a moisture retaining component, water in an amount which is in balance with the moisture retaining component (i.e., moisture of an amount which it is assumed that the moisture retaining component will, in equilibrium, retain and/or absorb from the environmental atmosphere), and a gelling agent. Depending on the Example, a foaming agent, an acid, or components improving the feel are additionally compounded.

In Tables 1 and 2, Comparative Examples 1 and 6 are examples in which a moisture retaining component and water in an amount which is in balance with the moisture retaining component (i.e., moisture of an amount which it is assumed that the moisture retaining component will, in equilibrium, retain and/or absorb from the environmental atmosphere) are compounded, but no gelling agent is compounded. Comparative Examples 2 and 7 are examples in which a moisture retaining component, water, and a polysaccharide which does not have a gelling function are compounded. Comparative Examples 3 and 8 are examples in which a moisture retaining component, water, and a polyglycerin unsaturated fatty acid ester which does not have a gelling function are compounded. Comparative Example 4 is an example in which only a wax (paraffin) is impregnated. Comparative Examples 5 and 9 are examples testing untreated samples (a base paper in Comparative Example 5 and a non-woven fabric in Comparative Example 9) for the purpose of comparison.

In Table 3, Examples 16 and 17 are examples in which sodium hydrogencarbonate (16-A and 17-A) is contained in the gel composition as a foaming agent, and lactic acid (16-B and 17-B) is contained in the gel composition as an acid. Further, Examples 18 and 19 are examples in which sodium hydrogencarbonate (18-A and 19-A) is contained in the gel composition as a foaming agent, and a lactic acid aqueous solution is used as the acid.

In Table 3, Examples 20 and 21 are examples in which silk powder is compounded. Examples 22 and 23 are examples using a base paper in which cross-linked pulp fibers are compounded. Comparative Example 10 is an example in which a paper, in which cross-linked pulp fibers are compounded in a base paper, is not treated and is used as the sample.

Further, in the respective Examples and Comparative Examples of Tables 1, 2 and 3, "external appearance" means the state at 20 DEG C, and for those Examples and Comparative Examples containing a gelling agent, means the state as viewed at 20DEG C after having been cooled once to the gel freezing point or below.

Details of the respective components shown in Tables 1, 2, 3 are as follows.
glycerin: "food additive glycerin (99% or more)", manufactured by Sakamoto Yakuhin Kogyo Co., Ltd.
sorbitol: "sorbitol SP", manufactured by Nikken Chemicals Co., Ltd.
polyethylene glycol 200: "PEG 200", manufactured by Dai-ichi Kogyo Seiyaku Co., Ltd.
agar powder: "kanten powder" manufactured by Ina Food Industry Co., Ltd.
hexaglycerin monostearate: "SY-Glyster MS 500" manufactured by Sakamoto Yakuhin Kogyo Co., Ltd.
hexaglycerin sesquistearate: "SY-Glyster SS500" manufactured by Sakamoto Yakuhin Kogyo Co., Ltd.
stearic acid: "Adeka fatty acid SA-910" manufactured by Asahi Denka Co., Ltd.
triethanol amine: "triethanol amine" manufactured by Mitsui Chemicals, Inc.
polyether modified silicone: "silicone KF354" manufactured by Shin-Etsu Chemical Co., Ltd.
sodium alginate: "kimitsualgin" manufetured by Kimitsu Chemical Industries Co., Ltd.
hexaglycerin monooleate: "SY-Glyster MO500" manufactured by Sakamoto Yakuhin Kogyo Co., Ltd.
paraffin (melting point 46-48 DEG C): manufactured by Junsei Chemical Co., Ltd.
sodium hydrogencarbonate: manufactured by Tosoh Corporation
lactic acid: manufactured by Purac Japan KK
silk powder: manufactured by Ueda Sen'i Kagaku Shinkokai (Ueda Fiber Science Promotion Society)

The items to be measured and the methods of measurement in Examples 1 through 23 and Comparative Examples 1 through 10 are as follows.

### A. Function Tests

### 1) Smoothness (Examples 1-10, 16-23, Comparative Examples 1-5, 10)

Each of the samples was touched by hand, and the tactile sensation was evaluated as follows.
extremely smooth: 4 points
smooth: 3 points
somewhat smooth: 2 points
not smooth: 2 point

The numbers of points obtained from evaluations by a panel of 10 persons were totaled, and the total number of points was given a mark as follows. (The same holds for the other evaluations described hereinafter.)
36 to 40 points: o &cir&
26 to 35 points: &cir&
16 to 25 points: DELTA
10 to 15 points: X

### 2) Pliability (Examples 1-23, Comparative Examples 1-10)

Each of the samples was touched by hand, and the tactile sensation was evaluated as follows.
extremely pliable: 4 points
pliable: 3 points
somewhat pliable: 2 points
not pliable: 1 point

### 3) Downiness (Examples 1-23, Comparative Examples 1-10)

Each of the samples was touched by hand, and the tactile sensation was evaluated as follows.
extremely downy: 4 points
downy: 3 points
somewhat downy: 2 points
not downy: 1 point

### 4) Skin Protecting/Moisturizing Sensation (Examples 11-15, Comparative Examples 6-9)

After the person conducting the evaluation washed their hand with tap water, they wiped the water off by using the sample, and evaluated the smoothness of their skin after wiping.
skin felt smooth: 3 points
skin felt somewhat smooth: 2 points
skin did not feel smooth: 1 point

### B. Measurement of Amount of Paper Dust (Examples 1-10, 16-23, Comparative Examples 1-5, 10)

Light was illuminated in a dark room by an incandescent lamp. The amount of paper dust generated when each sample was shook for the same amount of time (5 seconds) above the light path of the incandescent lamp was visually confirmed.
little paper dust: 3 points
somewhat large amount of paper dust: 2 points
large amount of paper dust: 1 point

### C. Physical Tests (Examples 1-10, 16-23, Comparative Examples 1-5, 10)

### 1) Tensile Strength

In accordance with the tensile strength test for tissue paper stipulated in JIS S-3104, the longitudinal direction strength of the paper when dry was measured. Measurement was carried out 10 times, and the average value thereof was determined.

### 2) Water Absorption Degree

The water absorption degree was measured in accordance with the water absorption degree test for tissue paper stipulated in JIS S-3104. Measurement was carried out 5 times, and the average value thereof was determined. Note that the JIS standard is 8 seconds or less.

The results of the respective tests are shown in Tables 4, 5 and 6.

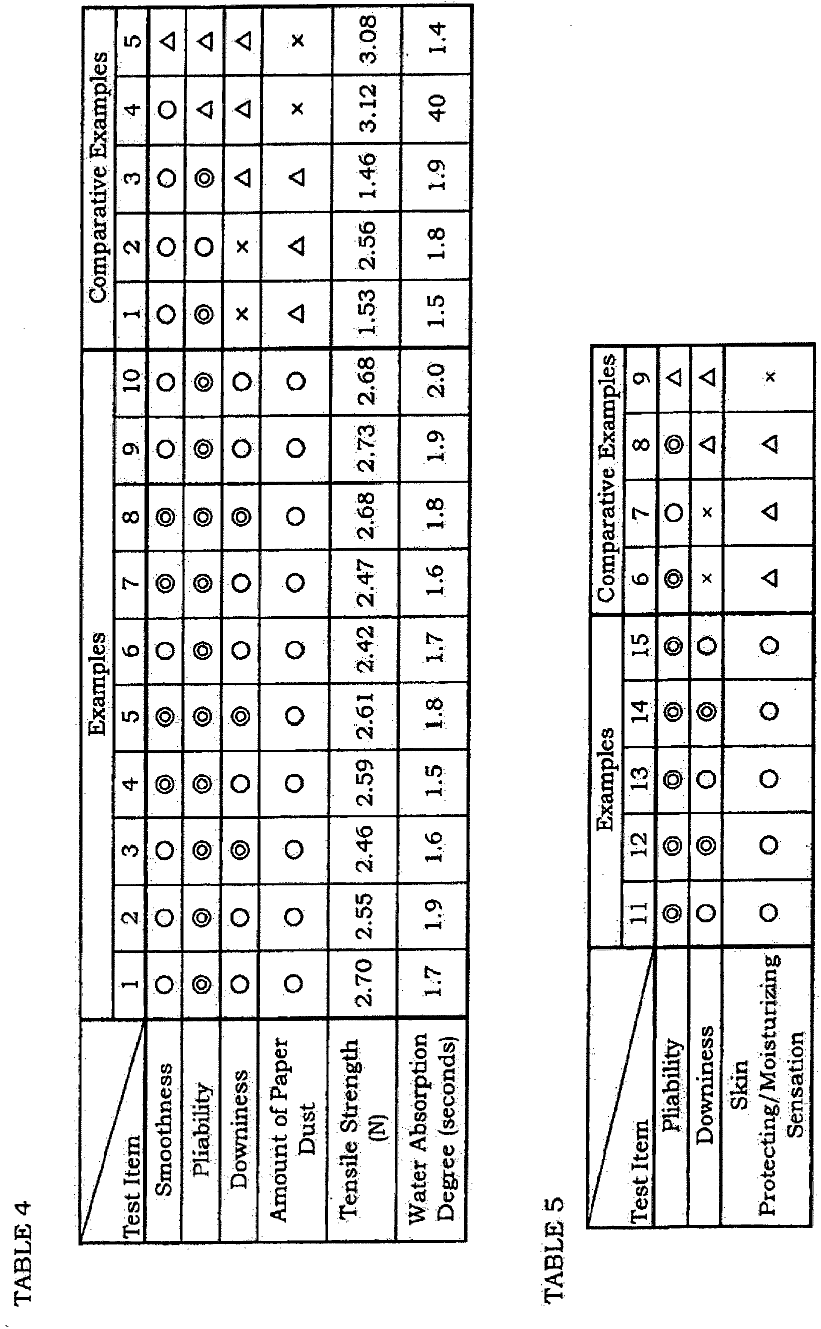

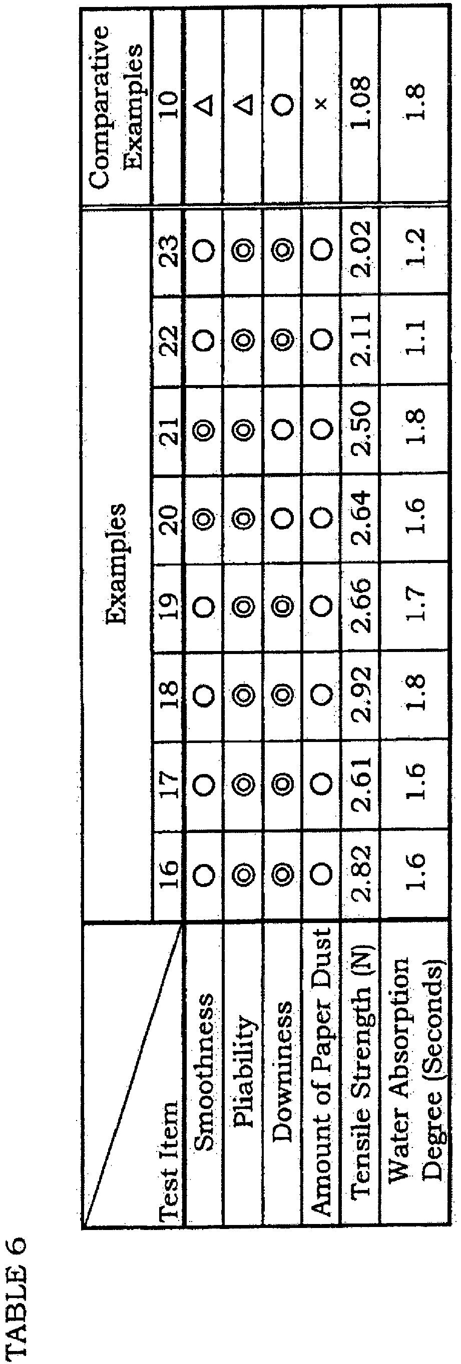

Table 4 shows the results of the tests on Examples 1 through 10 and Comparative Examples 1 through 5 which were listed in Table 1. Table 5 shows the results of the tests on Examples 11 through 15 and Comparative Examples 6 through 9 which were listed in Table 2. Table 6 shows the results of the tests on Examples 16 through 23 and Comparative Example 10 which were listed in Table 3.

In accordance with Examples 1 through 10 of Table 4, by including, in abase paper for sanitary paper, a water-containing gel composition which contains a moisture retaining component, a gelling agent and water, the evaluations of the smoothness, pliability, downiness and amount of paper dust of the fibrous web were all intermediate levels or better. As compared with Comparative Example 4 and Comparative Example 5 which was not subjected to any processing at all, it was confirmed that it was possible to obtain a fibrous web product which has a good balance of high-quality, cloth-like tactile sensations and which generates only a small amount of paper dust.

Moreover, when the amount of moisture contained in a fibrous web is large, there is a tendency for a sticky sensation to arise. However, by making the gel composition contain moisture as in Examples 1 through 10, such a sticky sensation is suppressed, and a downy feel can be obtained.

In Examples 3 and 5, both a saturated fatty acid and a salt thereof were used, and in Example 8, both agar and polyglycerin saturated fatty acid ester, which served as gelling agents, were used. The downiness was thereby improved in both cases. In Examples 4, 5, 7 and 8, it was confirmed that, by using a silicone as well, the smoothness was improved even more.

On the other hand, in Comparative Example 2, because the viscosity of the processing liquid increased without a gel being formed, the pliability was lacking and the downiness did not improve. Moreover, in Comparative Example 3, although a polyglycerin fatty acid ester was used, a gel was not formed and the evaluation of downiness was poor.

With regard to the tensile strength and the water absorbency as well, as compared with Comparative Example 5 which was untreated, in Examples 1 through 10, excellent characteristics were obtained, and it was confirmed that it was possible to obtain strength and water absorbency of a level sufficient in practical use for a fibrous web for sanitary use such as tissue paper, toilet paper or the like.

In accordance with Examples 11 through 15 of Table 5, by making a non-woven fabric contain a water-containing gel composition which contains a moisture retaining component, a gelling agent and water, the highest evaluations with respect to pliability could be obtained, in the same way as in Examples 1 through 10. In particular, in the example in which there was combined use of a silicone and a saturated fatty acid and salt thereof (Example 12) and in the example in which there was combined use of a silicone and polyglycerin saturated fatty acid ester, the downiness was improved. Moreover, it was confirmed that, in all of Examples 11 through 15, the downiness and the skin protecting/moisturizing sensation were superior to those of Comparative Examples 6 through 9.

In accordance with Examples 16 through 19 of Table 6, the downiness in particular was improved even more by generating, in the fibrous web, the foamed gel composition having a finely porous structure in the interior thereof. Further, in Examples 20 and 21 in which silk powder was compounded into the gel, the evaluation of the smoothness was high. In Examples 22 and 23 in which cross-linked pulp fibers were compounded into the base paper, it was confirmed that the base paper and the bulkiness of the cross-linked pulp fibers together result in an even greater improvement in the downiness.

In accordance with the present invention, there is provided a fibrous web product which is suited to sanitary (hygienic) uses, and which, while maintaining properties such as sufficient strength in actual use, water absorbing ability, water retaining ability and the like, also stably realizes a tactile sensation and feel which balance smoothness, pliability, downiness and the like which heretofore were unable to be realized in conventional fibrous web products, and which has excellent properties of low irritability to skin and the effect to protect and moisturize the skin, and which has particularly excellent downiness and smoothness without a sticky sensation or a greasy feeling. Moreover, in accordance with the present invention, there is also provided a method of manufacturing this fibrous web product. Further, the generation of paper dust by a product using paper as the fibrous web can be suppressed.

The features disclosed in the foregoing description, in the claims and/or in the accompanying drawings may, both separately and in any combination thereof, be material for realising the invention in diverse forms thereof.

## Claims

1. A fibrous web product in which a water-containing gel composition is contained in a fibrous web, wherein the water-containing gel composition contains a moisture retaining component, a gelling agent, and water, and the water is at least one of water retained by the moisture retaining component and water absorbed from the atmosphere by the moisture retaining component,
**characterized in that**
the moisture retaining component is glycerin and
the gelling agent is at least one type selected from the group consisting of hexaglycerin monostearate and agar.

2. The fibrous web product of claim 1, wherein the water-containing gel composition is thermally reversible, such that it is set in a gel state by being cooled and is set in a sol state by being heated.

3. The fibrous web product of any of claims 1 and 2, wherein the water-containing gel composition further contains silk powder.

4. The fibrous web product of any of claims 1 through 3, wherein the water-containing gel composition is a foamed gel composition having a finely porous structure.

5. The fibrous web product of claim 4, wherein the foamed gel composition which has the finely porous structure is formed by foaming of the gas being generated due to reaction of the foaming agent and the acid when the gel composition containing a foaming agent is made to contact an acid.

6. The fibrous web product of claim 4, wherein the foamed gel composition which has the finely porous structure is formed by foaming of the gas being generated by heating a gel composition containing a foaming agent and decomposing the foaming agent.

7. The fibrous web product of claim 5 or 6, wherein the foaming agent is at least one type selected from the group consisting of sodium hydrogencarbonate, ammonium carbonate, azodicarbonamide, and benzene sulfonyl hydrazide.

8. The fibrous web product of any of claims 1 through 7, wherein the fibrous web contains pulp fibers, and the pulp fibers contain cross-linked pulp fibers in an amount of from 1 % by mass to 80 % by mass.

## Patentansprüche

1. Faservliesprodukt, wobei eine Wasser enthaltende Gelzusammensetzung in einem Faservlies enthalten ist, wobei die Wasser enthaltende Gelzusammensetzung eine feuchtigkeitsspeichernde Komponente, einen Gelierstoff und Wasser enthält und das Wasser mindestens eines von durch die feuchtigkeitsspeichernde Komponente gespeichertes Wasser und durch die feuchtigkeitsspeichernde Komponente aus der Atmosphäre absorbiertes Wasser ist,
**dadurch gekennzeichnet, dass**
die feuchtigkeitsspeichernde Komponente Glyzerin ist und
der Gelierstoff mindestens ein Typ ist, der aus der Gruppe ausgewählt ist, die aus Hexaglyzerinmonostearat und Agar besteht.

2. Faservliesprodukt nach Anspruch 1, wobei die Wasser enthaltende Gelzusammensetzung thermisch reversibel ist, sodass sie durch Kühlen in einen Gelzustand versetzt wird und durch Erwärmen in einen Solzustand versetzt wird.

3. Faservliesprodukt nach einem der Ansprüche 1 und 2, wobei die Wasser enthaltende Gelzusammensetzung ferner Seidenpuder enthält.

4. Faservliesprodukt nach einem der Ansprüche 1 bis 3, wobei die Wasser enthaltende Gelzusammensetzung eine geschäumte Gelzusammensetzung mit einer feinporösen Struktur ist.

5. Faservliesprodukt nach Anspruch 4, wobei die geschäumte Gelzusammensetzung, die die feinporöse Struktur aufweist, durch Schäumen des Gases, das, wenn die ein Schäummittel enthaltende Gelzusammensetzung mit einer Säure in Kontakt gebracht wird, auf Grund einer Reaktion des Schäummittels und der Säure erzeugt wird gebildet ist.

6. Faservliesprodukt nach Anspruch 4, wobei die geschäumte Gelzusammensetzung, die die feinporöse Struktur aufweist, durch Schäumen des Gases, das durch Erwärmen einer Gelzusammensetzung, die ein Schäummittel enthält, und Zersetzen des Schäummittels erzeugt ist, gebildet ist.

7. Faservliesprodukt nach Anspruch 5 oder 6, wobei das Schäummittel mindestens ein Typ ist, der aus der Gruppe ausgewählt ist, die aus Sodiumhydrogencarbonat, Ammoniumcarbonat, Azodicarbonamid und Benzensulfonylhydrazid besteht.

8. Faservliesprodukt nach einem der Ansprüche 1 bis 7, wobei das Faservlies Zellstofffasern enthält und die Zellstofffasern vernetzte Zellfasern in einer Menge von 1 Gewichtsprozent bis 80 Gewichtsprozent enthalten.

## Revendications

1. Produit de voile fibreux dans lequel une composition de gel contenant de l'eau est contenue dans un voile fibreux, dans lequel la composition de gel contenant de l'eau contient un composant de rétention d'humidité, un agent gélifiant, et de l'eau, et l'eau est au moins de l'eau parmi de l'eau retenue par le composant de rétention d'humidité et de l'eau absorbée en provenance de l'atmosphère par le composant de rétention d'humidité,
**caractérisé en ce que**
le composant de rétention d'humidité est de la glycérine et
l'agent gélifiant est au moins un type sélectionné dans le groupe constitué par du monostéarate d'hexaglycérine et de l'agar-agar.

2. Produit de voile fibreux selon la revendication 1, dans lequel la composition de gel contenant de l'eau est thermiquement réversible, de sorte qu'elle est durcie dans un état de gel en étant refroidie et est durcie dans un état solide en étant chauffée.

3. Produit de voile fibreux selon l'une quelconque des revendications 1 et 2, dans lequel la composition de gel contenant de l'eau contient par ailleurs de la poudre de soie.

4. Produit de voile fibreux selon l'une quelconque des revendications 1 à 3, dans lequel la composition de gel contenant de l'eau est une composition de gel en mousse ayant une structure finement poreuse.

5. Produit de voile fibreux selon la revendication 4, dans lequel la composition de gel en mousse qui a la structure finement poreuse est formée en faisant mousser le gaz produit en raison de la réaction de l'agent moussant et de l'acide quand la composition de gel contenant un agent moussant est entraînée à entrer en contact avec un acide.

6. Produit de voile fibreux selon la revendication 4, dans lequel la composition de gel en mousse qui a la structure finement poreuse est formée en faisant mousser le gaz produit en chauffant une composition de gel contenant un agent moussant et en décomposant l'agent moussant.

7. Produit de voile fibreux selon la revendication 5 ou la revendication 6, dans lequel l'agent moussant est au moins un type sélectionné dans le groupe constitué par de l'hydrogénocarbonate de sodium, du carbonate d'ammonium, de l'azodicarbonamide, et du benzène-sulfonhydrazide.

8. Produit de voile fibreux selon l'une quelconque des revendications 1 à 7, dans lequel le voile fibreux contient des fibres de pâte à papier, et les fibres de pâte à papier contiennent des fibres de pâte à papier réticulées selon une quantité allant de 1 % en masse à 80 % en masse.
